# EUROPÄISCHE PATENTSCHRIFT

(11) **EP 2 820 950 B1**
(45) Veröffentlichungstag und Bekanntmachung des Hinweises auf die Patenterteilung: **08.03.2017**
(21) Anmeldenummer: 14002206.2
(22) Anmeldetag: 27.06.2014
(51) Int. Cl.: A01M 9/00, A01K 67/033

(54) **Ausbringfahrzeug zum Ausbringen von Nützlingsorganismen enthaltenden Ausbringeinheiten**
Dispersion vehicle for applying units containing beneficial organisms
Véhicule d'épandage pour épandre des unités d'épandage contenant des organismes utiles

(30) Priorität: 05.07.2013 DE 102013011234
(43) Veröffentlichungstag der Anmeldung: 07.01.2015
(73) Patentinhaber: Biocare Gesellschaft für Biologische Schutzmittel mbH, 37574 Einbeck (DE)
(72) Erfinder: Beitzen-Heineke, Wilhelm, 37574 Einbeck (DE); Peter, Bruno F., 37574 Einbeck (DE)
(74) Vertreter: Grättinger Möhring von Poschinger Patentanwälte Partnerschaft

(56) Entgegenhaltungen:
- EP-A1- 2 559 330
- DE-A1- 3 918 554
- DE-A1- 10 140 773
- FR-A1- 2 583 256
- FR-A1- 2 697 130
- US-A- 3 994 437
- US-A1- 2012 234 220

## Beschreibung

Die vorliegende Erfindung betrifft das großflächige Ausbringen von Ausbringeinheiten, welche zur biologischen Schädlingsbekämpfung geeignete Nützlingsorganismen enthalten, in einen land-, forst- oder gartenwirtschaftlichen Bestand. In größerem Detail betrifft die Erfindung das derartige Ausbringen von im Wesentlichen kugelförmigen Ausbringeinheiten mit einem mittleren Durchmesser zwischen 15mm und 30mm und einer mittleren Dichte zwischen 0,08g/cm³ und 1,5g/cm³.

Zur Bekämpfung von Schädlingen in land-, forst- oder gartenwirtschaftlichen Kulturflächen werden zunehmend biologische Verfahren eingesetzt, d.h. solche Verfahren, bei denen zur Bekämpfung der Schädlinge spezifische Nützlinge ausgebracht werden. Zur Ausbringung der Nützlinge in den betreffenden Bestand dienen dabei spezielle Behältnisse. Insoweit ist beispielsweise zu verweisen auf die DE 19624596 C2 und den in dieser Schrift diskutierten Stand der Technik sowie die EP 1161863 A1. Mehr oder weniger kugelförmige Ausbringeinheiten, um deren Ausbringung es vorliegend geht, sind bekannt zur Ausbringung von Nützlingen (vgl. EP 1161863 A1) und von vermehrungsfähigen Nützlingspopulationen (vgl. WO 2011/104002 A1). Auch für solche im Wesentlichen kugelförmigen Ausbringeinheiten ist in der Praxis die Einzelausbringung typisch, insbesondere indem mit Aufhängern versehene kapselartige Behältnisse an Pflanzen aufgehängt werden (EP 1161863 A1) bzw. Gebilde aus mehreren mittels einer Verbindungsschnur miteinander verbundenen Ausbringeinheiten von Hand über ausgewählte Pflanzen im Bestand gehängt werden (WO 2011/104002 A1).

Zwar erwähnt die EP 1161863 A1 u.a. das Abwerfen der dort offenbarten Kapseln aus Klein- oder Modellflugzeugen; und in der WO 2011/104002 A1 wird u.a. eine maschinelle Ausbringung vereinzelter kugelförmiger Ausbringeinheiten erwähnt. Eine solche hat sich bisher allerdings nicht realisieren lassen, schon gar nicht in kommerziell attraktiver Weise. Dies hängt unmittelbar mit den im Wesentlichen kugelförmigen Ausbringeinheiten selbst und deren charakteristischen Eigenschaften zusammen. So ist die Hülle der Ausbringeinheiten, damit diese ihren Zweck und ihre Funktion erfüllen können, typischerweise nicht sonderlich robust, sondern vielmehr vergleichsweise filigran und auf diese Weise eher empfindlich. Zudem sind die Ausbringeinheiten ausgesprochen leicht.

Der vorliegenden Erfindung liegt im Lichte der vorstehend dargelegten Situation die Aufgabe zugrunde, eine Möglichkeit bereitzustellen, mit welchem sich im wesentlichen kugelförmige, Nützlingsorganismen enthaltende Ausbringeinheiten der weiter oben dargelegten Art wirtschaftlich großflächig auf land-, forst- bzw. gartenwirtschaftlichen Beständen ausbringen lassen.

Gelöst wird diese Aufgabestellung gemäß der vorliegenden Erfindung durch das in Anspruch 1 angegebene Ausbringfahrzeug, welches mit einem erfindungsgemäß ausgeführten, weiter unten eingehend erläuterten Ausbringgerät ausgestattet ist und in Bahnen über den Boden des Bestands bewegt werden kann, wobei die Ausbringeinheiten einzeln getaktet mittels Druckluft aus seitwärts gerichteten, frei mündenden Ausblasrohren des Ausbringgeräts zu beiden Seiten des Fahrzeugs verschossen werden. Ein solches Verschießen der Ausbringeinheiten zu beiden Seiten eines Ausbringfahrzeugs (sowie ggfs. auch in dessen Spur; s.u.) ermöglicht es, den Bestand mit einer geringstmöglichen Anzahl an Überquerungen großflächig mit den hier in Rede stehenden im Wesentlichen kugelförmigen Ausbringeinheiten zu behandeln, wobei durch das einzeln getaktete Verschießen der Ausbringeinheiten ein hinreichend hohes Maß an Dosiergenauigkeit erreicht wird. Dies ist hinwiederum ausschlaggebend für ein hohes Maß an Wirtschaftlichkeit, weil sich auf diese Weise mit einer geringstmöglichen Anzahl an ausgebrachten Ausbringeinheiten die Nützlingsorganismen (unter Berücksichtigung von deren Ausbreitung um die jeweilige Ausbringeinheit herum) flächendeckend bereitstellen lassen. Im Falle einer landgestützten Ausbringung der Ausbringeinheiten (s.u.) durch deren seitwärts gerichtetes Verschießen vom Ausbringfahrzeug aus sind zudem die Schäden bzw. Ausfälle durch das Befahren des Bestands mit einem entsprechenden Fahrzeug sehr gering, namentlich auch Schäden durch das Wenden des Ausbringfahrzeugs am Ende der jeweiligen Bahn. Denn trotz der ungünstigen Flugeigenschaften der hier in Rede stehenden Ausbringeinheiten, wie sie die Folge eines vergleichsweise großen Volumens bei sehr geringem Gewicht sind, lassen sich in Anwendung der vorliegenden Erfindung mit geringem apparativen Aufwand selbst bei einer landgestützten Ausbringung solche Wurfweiten realisieren, dass der Abstand zwischen den einzelnen zu befahrenden Bahnen beispielsweise 24m betragen kann. Für solche Wurfweiten ist noch nicht einmal ein apparativer Aufwand für ein Ausbringgestänge oder dergleichen erforderlich.

Unter Gesichtspunkten der Wirtschaftlichkeit ist hervorzuheben, dass landgestützte Ausbringfahrzeuge sich mit einer vergleichsweise hohen Fortbewegungsgeschwindigkeit über die zu behandelnden Flächen bewegen können, bei typischen Untergrund- und Anwendungsverhältnissen beispielsweise mit einer Geschwindigkeit zwischen 25km/h und 35km/h. Dies lässt die biologische Schädlingsbekämpfung mit einer sehr großen, bisher nicht realisierbaren Hektarleistung (z.B. 50ha/h) zu. Ersichtlich noch wesentlich höhere Hektarleistungen lassen sich bei einer luftgestützten Anwendung, d.h. unter Einsatz eines erfindungsgemäßen Luftfahrzeugs, erzielen, nachdem als Ausbringfahrzeug dienende Luftfahrzeuge (z.B. Helikopter oder Ultraleichtflugzeuge) sich mit Geschwindigkeiten bis zu 80km/h oder sogar noch darüber über den Bestand bewegen können, ohne dass die zuverlässige Ausbringung der Ausbringeinheiten gefährdet würde.

Hervorzuheben ist dabei, dass das getaktete Verschießen der hier in Rede stehenden relativ großvolumigen und leichten kugelförmigen Ausbringeinheiten mittels Druckluft aus seitwärts gerichteten Ausblasrohren, wie es für großflächige Ausbringen der Ausbringeinheiten in praktischer Umsetzung der vorliegenden Erfindung charakteristisch ist, überhaupt erst durch die Bereitstellung eines hierfür geeigneten Ausbringgeräts ermöglicht wird, wie dieses - als Bestandteil eines Ausbringfahrzeugs - die vorliegende Erfindung ausmacht (s.u.). Vorschläge wie das Verteilen von pulver- oder granulatförmigem Material aus der Luft (vgl. US 3484062 A) sind im Zusammenhang mit den hier maßgeblichen Ausbringeinheiten bereits unter technischen Gesichtspunkten nicht umsetzbar. Entsprechendes gilt für landwirtschaftliche Maschinen, nämlich Drillmaschinen wie solche gemäß der US 2012/0234220 A1, welche Samenkörner (auf der Arbeitsbreite der Drillmaschine verteilt) über Drillrohre in mehreren Drillfurchen ablegen, wobei der Abstand der Samenkörner zueinander in der jeweiligen Drillfurche über eine pneumatische, eine sich drehende gelochte Trommel aufweisende Vereinzelungseinrichtung vorgegeben wird. Dokument FR 2 583 256 A1 offenbart ein Ausbringfahrzeug für das großflächige Ausbringen von im Wesentlichen kugelförmigen, zur biologischen Schädlingsbekämpfung geeignete Nützlingsorganismen enthaltenden Ausbringeinheiten gemäß dem Oberbegriff des unabhängigen Anspruchs 1.

Das erfindungsgemäße Ausbringfahrzeug umfasst ein Ausbringgerät mit einem Gehäuse, einem Vorratsraum für die Ausbringeinheiten und mehreren Ausblasrohren für die auszubringenden Ausbringeinheiten, welche strömungstechnisch an den Innenraum des Gehäuses angeschlossen sind und von denen zumindest zwei zu unterschiedlichen Seiten des Fahrzeugs orientiert münden, wobei mindestens ein Druckluftgebläse vorgesehen ist, das aus der Umgebung angesaugte Luft kontinuierlich durch das Gehäuse und mit mindestens 20m/s (bevorzugt mit mehr als 40m/s) durch die Ausblasrohre fördert. Die Einheit aus Gehäuse und Vorratsraum für die Ausbringeinheiten ist dabei - abgesehen von den für die permanente Durchströmung mit Druckluft erforderlichen Öffnungen (Ansaug- und Austrittsöffnungen) - im Wesentlichen druckdicht, so dass die Förderleistung des Gebläses im größtmöglichen Umfang für das Verschießen der Ausbringeinheiten zur Verfügung steht. Je nach der technischen Umsetzung der getakteten Zufuhr der Ausbringeinheiten zu den Ausblasrohren wird das Gehäuse des Ausbringgeräts samt dem Vorratsraum für die Ausbringeinheiten ständig auf einem mehr oder weniger hoch (mindestens 5mbar) über dem Umgebungsdruck liegenden Druckniveau gehalten (s.u.). Indem das Gehäuse auf diese Weise - bei einer kontinuierlichen Durchströmung von Gehäuse und Ausblasrohren mit Druckluft - unter einem Überdruck gehalten wird, können die Ausbringeinheiten ohne ausgeprägte Druckluftimpulse verschossen werden. Dabei sind die Löcher in den einzelnen Lochreihen der Trommel zueinander versetzt angeordnet. Auf diese Weise werden die Ausbringkugeln durch die Ausblasrohre hindurch aufeinander abfolgend verschossen. Hierdurch lassen sich nicht nur die Druckverhältnisse innerhalb des Gehäuses vergleichmäßigen, d.h. nennenswerte Druckschwankungen und insbesondere Druckpulsationen, welche für die hier in Rede stehenden Ausbringkugeln schädlich wären, vermeiden. Zudem ist auch die Verteilung der Ausbringkugeln im Bestand positiv beeinflusst, so dass mit einer minimalen Ausbringdichte gearbeitet werden kann, was der Wirtschaftlichkeit entgegenkommt. Die Ausbringeinheiten werden, mit anderen Worten, im Bereich der Eintrittsöffnung des jeweiligen Ausblasrohres an den dieses kontinuierlich durchströmenden Luftstrom übergeben. Dies ist ein maßgeblicher Aspekt für das schadlose Verschießen der hier in Rede stehenden kugelförmigen Ausbringeinheiten; denn ausgeprägte Druckluftimpulse würden eine Beschädigung der Hülle der Ausbringeinheiten nach sich ziehen bzw. deren Integrität gefährden. Insoweit spielt auch eine Rolle, dass die Ausbringeinheiten typischerweise nicht vollständig geschlossene (ballartige) Kugeln sind, sondern vielmehr speziell dimensionierte Schlupflöcher für die Nützlinge aufweisen, welche nicht beschädigt werden dürfen.

Das im Rahmen der Erfindung eingesetzte Ausbringgerät weist eine Einrichtung zum Vereinzeln der Ausbringeinheiten und zu deren getakteter Zufuhr zu den Ausblasrohren auf. Diese Vereinzelungseinrichtung umfasst eine drehend angetriebene (bevorzugt stirnseitig gegenüber dem Gehäuse abgedichtete) Trommel mit sich in Umfangsrichtung erstreckenden Reihen von zueinander beabstandeten Löchern und eine innen an der Trommel anliegende, nicht-drehende Abdeckeinrichtung für das Verschließen der Löcher über einen vorgegebenen Winkelbereich des Umlaufs der Trommel. Eine solche Ausführung der Vereinzelungseinrichtung ist darauf abgestimmt, dass das Gebläse - abgestimmt auf die übrigen Komponenten des Ausbringgeräts - zur Erzeugung eines Überdrucks in dem Gehäuse geeignet ist, beispielsweise von mindestens 5mbar. Innen ist die Trommel dabei (durch Belüftung) einem geringeren als dem im Gehäuse herrschenden Überdruck ausgesetzt, insbesondere dem Umgebungsdruck oder einem zwischen diesem und dem Gehäuse-Überdruck liegenden Druck (s.u.). Durch das Druckgefälle zwischen jenem Innenraum des Gehäuses, in dem sich die Ausbringkugeln befinden, und dem Innenraum der Trommel legen sich die im Wesentlichen kugelförmigen Ausbringeinheiten ("Ausbringkugeln") im Bereich der Löcher der Trommel an Letzterer an und werden auf diese Weise den Eintrittsöffnungen der Ausblasrohre zugeführt. Die Abdeckeinrichtung verschließt die in der Trommel vorgesehenen Löcher auf dem Teil des TrommelUmlaufs von der Übergabe der Ausbringeinheiten an die Ausblasrohre bis zu dem Bereich des Vorratsraumes, in dem die bevorrateten Ausbringeinheiten zur Anlage an die Trommel bereitgehalten werden. Durch das Verschließen der Löcher der Trommel benachbart den Eintrittsöffnungen der Ausblasrohre lässt sich das Anhaften der Ausbringeinheiten an der Trommel mindern, was die beschädigungsfreie Übergabe der empfindlichen Ausbringeinheiten an die Ausblasrohre begünstigt. Und das fortgesetzte Verschließen der Löcher der Trommel im weiteren Verlauf des Trommelumlaufs verhindert ein Entweichen der Druckluft durch die Trommel hindurch. Namentlich in Ansehung der (für den zuverlässigen Transport der Ausbringeinheiten vor die Eintrittsöffnungen der Ausblasrohre) bevorzugten Dimensionierung der Löcher der Trommel (s.u.) ist das Verschließen der Löcher mittels der besagten Abdeckeinrichtung auch ein Aspekt der Wirtschaftlichkeit. Oder mit anderen Worten: Durch die besagte Abdeckeinrichtung ist ein gleichermaßen wirtschaftlicher und zuverlässiger Betrieb des Ausbringgeräts im Zusammenhang mit den hier in Rede stehenden Ausbringeinheiten möglich. Nur der Vermeidung von Missverständnissen halber wird darauf hingewiesen, dass die Angabe, wonach die Abdeckeinrichtung innen an der Trommel anliegt, keineswegs dahingehend interpretiert werden darf, dass die Abdeckeinrichtung ausschließlich innen an der Trommel anliegt. Vielmehr kann im Rahmen der vorliegenden Erfindung die Abdeckeinrichtung - um ihre Funktion, die Löcher der Trommel über einen Teil des Umlaufs der Trommel zu verschließen, zu erfüllen - durchaus sowohl innen als auch außen an der Trommel anliegen. Insbesondere in Nachbarschaft zu den Eintrittsöffnungen der Ausblasrohre empfiehlt sich aber, die Abdeckeinrichtung innen an der Trommel anliegen zu lassen, weil sich dies positiv auf die Integrität der Ausbringeinheiten auswirkt.

Gemäß einer bevorzugten Weiterbildung der Erfindung umfasst die Abdeckeinrichtung eine ruhende Innentrommel in Form einer Steuerhülse, die über einen Teil des Umfangs in Umfangsrichtung sich erstreckende, zu den Lochreihen der Trommel fluchtende Schlitze aufweist. Auf diese Weise ist mit geringem Aufwand eine zuverlässige Abdeckung der Löcher in der Trommel über einen großen Winkelbereich möglich, was im Falle relativ groß dimensionierter Löcher der Trommel ein wichtiger Aspekt für sowohl die Wirtschaftlichkeit als auch den apparativen Aufwand ist. Denn je mehr Druckluft das Gehäuse durch die Trommel hindurch und deren Belüftung verlässt, desto geringer ist die Effizienz des Ausbringgeräts und desto größer müssen die Druckluftgebläse ausgelegt werden, bei einer entsprechend höheren Leistungsaufnahme. Im Lichte dieser Zusammenhänge ist die vorstehend beschriebene, als ruhende Innentrommel ausgeführte Abdeckeinrichtung ideal.

Die in der Trommel der Vereinzelungseinrichtung vorgesehenen Löcher weisen gemäß einer abermals anderen bevorzugten Weiterbildung der Erfindung einen Durchmesser auf, der zwischen 45% und 75%, besonders bevorzugt zwischen 50% und 70% des mittleren Durchmessers der Ausbringkugeln beträgt. Bei üblich dimensionierten Ausbringkugeln liegt der Durchmesser der Löcher demnach typischerweise zwischen 7mm und 20mm. Eine solche Abstimmung ist im Hinblick auf die Natur und Eigenschaften der hier in Rede stehenden Ausbringkugeln günstig, um deren Beschädigung beim Vereinzeln an der Trommel durch das im Bereich der Löcher der Trommel bestehende Druckgefälle zu unterbinden. Insoweit ist zu bedenken, dass typischerweise ein durchaus nennenswertes Druckgefälle (z.B. etwa 10mbar) vorliegt, weil zum Verschießen der Ausbringkugeln mit ausreichend hoher Mündungsgeschwindigkeit ein nicht unerheblicher Überdruck innerhalb des Gehäuses herrschen muss. Auf der anderen Seite stellt die vorliegende Dimensionierung der Löcher der Trommel hinreichend sicher, dass die Ausbringkugeln zuverlässig zu den Eintrittsöffnungen der Ausblasrohre transportiert werden und nicht auf dem Weg dorthin von der Trommel abfallen, insbesondere bei den typischerweise beim Überfahren von unebenen landwirtschaftlichen Flächen auftretenden Erschütterungen des Ausbringfahrzeugs.

Die in der Trommel vorgesehenen Löcher können eine mehr oder weniger an die Form der Ausbringeinheiten angepasste (z.B. leicht konische) Gestalt haben. Dies ist indessen keineswegs zwingend. In diesem Zusammenhang spielt auch die Wandstärke der Trommel eine Rolle. Namentlich bei einer (das Anhaften der Kugeln an der Trommel auch bei einem eher geringen Druckgefälle begünstigenden) vergleichsweise großen Dimensionierung der Löcher (z.B. im Bereich von 70-80% des Durchmessers der Ausbringkugeln) wird die Wandstärke der Trommel bevorzugt größer gewählt als dies allein unter statischen Gesichtspunkten erforderlich wäre, damit die Ausbringkugeln im Bereich der Löcher nicht allzu weit in den Innenraum der Trommel vorstehen. So kann die Gefahr, dass die Ausbringkugeln beim Auftreffen auf die Abdeckeinrichtung ggf. beschädigt werden, reduziert werden. Auf der anderen Seite kann sich in gewissem Umfang durchaus als nützlich erweisen, wenn die (leicht in das Innere der Trommel hinein ragenden) Ausbringkugeln, wenn sie vor die Eintrittsöffnungen der Ausblasrohre gelangen, durch die Abdeckeinrichtung mechanisch von der Trommel abgehoben und ggf. sogar mehr oder weniger ausgeprägt in Richtung auf das jeweilige Ausblasrohr hin gestoßen werden.

Für typische Anwendungsfälle der landgestützen, d.h. mittels Landfahrzeugen erfolgenden Ausbringung der Ausbringeinheiten weist jede Lochreihe der Trommel ein bis sechs Löcher, bevorzugt zwei bis vier Löcher auf. Bei dieser Anzahl von Löchern ergibt sich ein für den zuverlässigen Transport der Ausbringeinheiten bevorzugter Drehzahlbereich der Trommel (bevorzugt 1-10 Umdrehungen pro Minute, besonders bevorzugt 3-7 Umdrehungen pro Minute). Für luftgestützte Anwendungsfälle kommen demgegenüber bevorzugt Trommeln mit einer größeren Anzahl an Löchern zum Einsatz, beispielsweise zwei bis zwölf Löcher, bevorzugt vier bis acht Löcher pro Lochreihe. Dies ermöglicht eine höhere Frequenz für den Abschuss der Ausbringeinheiten ohne substantielle Erhöhung der Drehzahl der Trommel.

Ebenfalls zum Schutz der im Wesentlichen kugelförmigen Ausbringeinheiten vor einer Zerstörung bzw. Beschädigung der Hülle ist vorteilhaft, wenn der Durchmesser der Ausblasrohre zwischen 5% und 20% über dem Durchmesser der im Wesentlichen kugelförmigen Ausbringeinheiten liegt. Auf diese Weise verschließen die kugelförmigen Ausbringeinheiten die Ausblasrohre nicht vollständig; vielmehr ist in einem gewissen Umfang eine Umströmung möglich. Zerstörerische bzw. zumindest die Ausbringkugeln unzulässig verformende Druckunterschiede auf beiden Seiten der Ausbringkugeln werden auf diese Weise unterbunden.

Bei solchen Ausbringgeräten, die für besonders große Arbeitsbreiten ausgeführt sind, kann gemäß einer abermals anderen bevorzugten Weiterbildung der vorliegenden Erfindung vorgesehen sein, dass in dem Innenraum der Trommel nicht - durch eine entsprechende Belüftung - der Umgebungsdruck herrscht, sondern vielmehr ein zwischen diesem und dem Überdruck im Gehäuse liegender Druck, zu welchem Zweck der Innenraum der Trommel über ein Druckbegrenzungsventil belüftet ist. Auf diese Weise wird die Druckdifferenz zwischen dem Innenraum des Gehäuses und dem Inneren der Trommel unter dem für das Verschießen der Ausbringkugeln maßgeblichen Druckgefälle gehalten, so dass die Ausbringkugeln besonders weit verschossen werden können, ohne dass die Gefahr einer Beschädigung der Ausbringkugeln durch ein unzulässig hohes Druckgefälle an den Löchern der Trommel der Vereinzelungseinrichtung besteht.

Wenngleich für typische Anwendungsfälle Ausbringgeräte mit drei Ausblasrohren generell besonders geeignet sind, können bei besonders gelagerten Fällen auch mehr Ausblasrohre zweckmäßig sein, beispielsweise fünf oder sieben. Auf diese Weise lassen sich ggf. noch größere Arbeitsbreiten erreichen als mit drei Ausblasrohren, beispielsweise mit Bahnabständen von 40m (bei fünf Ausblasrohren), wobei die seitwärts gerichteten Ausblasrohre entsprechend lang ausgeführt sind. Hier erweist sich eine Unterstützung mittels eines Gestänges als nützlich. Im Übrigen sind solche Ausbringgeräte mit einer höheren Anzahl von Ausblasrohren (wegen der erforderlichen höheren Druckluftdurchsätze und Drücke) ein Fall für die vorstehend angesprochene gedrosselte Belüftung der Trommel.

Gemäß einem anderen vorteilhaften Aspekt der vorliegenden Erfindung ist unterhalb der Trommel der Vereinzelungseinrichtung innerhalb des Gehäuses ein vom Druckluftgebläse her mit Luft durchströmtes Lochblech angeordnet. Dies begünstigt eine ständige Bewegung der Ausbringkugeln in dem betreffenden Bereich in Art eines Wirbelbetts, was hinwiederum günstig ist für ein zuverlässiges Anlagern der Ausbringkugeln an der Trommel der Vereinzelungseinrichtung. Um die Gefahr, dass an einem Loch der Trommel zwei Ausbringkugeln gemeinsam befördert werden, zu reduzieren, können den Lochreihen Abstreifer (z.B. in Form von Elastomerstäben) zugeordnet sein, welche so positioniert sind, dass beim Passieren eines doppelt belegten Loches eine der beiden Ausbringkugeln an dem Abstreifer anstößt und entweder selbst abgestreift wird, so dass sie in den Vorrat zurückfällt, oder aber direkt vor das betreffende Loch der Trommel geschoben wird, so dass die andere der beiden Ausbringkugeln in den Vorrat zurückfällt.

Eine andere bevorzugte Weiterbildung der vorliegenden Erfindung zeichnet sich dadurch aus, dass die Trommel durch einen Gehäusedurchbruch hindurch ohne Zerlegen des Gehäuses auswechselbar ist. Bevorzugt ist eine die Trommel und die Abdeckeinrichtung umfassende, in Art einer Kartusche ausgeführte Einheit durch den Gehäusedurchbruch hindurch auswechselbar. Auf diese Weise kann mit minimalem Aufwand das Ausbringgerät an unterschiedliche Aufgaben angepasst werden, indem eine durch einen ersten Lochabstand charakterisierte Trommel gegen eine andere Trommel, bei welcher die Löcher einen anderen Lochabstand aufweisen, ausgetauscht wird. Auch im Hinblick auf die Anpassung des Ausbringgeräts an unterschiedliche Ausbringkugeln ist diese Weiterbildung vorteilhaft, weil Trommeln mit unterschiedlichen Lochdurchmessern einfach und ohne nennenswerten Aufwand gegeneinander ausgetauscht werden können.

Gemäß einer abermals anderen bevorzugten Weiterbildung der vorliegenden Erfindung ist vorgesehen, dass mindestens einem der Ausblasrohre eingangsseitig ein von einem Steuergerät aus betätigbarer Verstellmechanismus zugeordnet ist. Auf diese Weise lassen sich einzelne der Ausblasrohre vorübergehend außer Betrieb setzen bzw. "stilllegen", um die Arbeitsbreite des Ausbringgeräts an die örtlichen Gegebenheiten anpassen zu können, beispielsweise am Rand der jeweils zu bearbeitenden landwirtschaftlichen Fläche. Alternativ oder ggf. zusätzlich ist denkbar, die Abdeckeinrichtung dergestalt verstellbar auszuführen, dass ausgewählte Lochreihen während des vollständigen Trommelumlaufs verschlossen bleiben, so dass in der betreffenden Lochreihe eine Förderung von Ausbringeinheiten unterbleibt.

Das im Rahmen der vorliegenden Erfindung einsetzbare Ausbringgerät bzw. das mit diesem ausgestattete Fahrzeug kann noch über eine Reihe weiterer Aspekte in vorteilhafter Weise weitergebildet sein, beispielsweise im Hinblick auf eine erhöhte Betriebssicherheit und/oder Bedienerfreundlichkeit. So kann beispielsweise eine Kontrollvorrichtung vorgesehen sein, welche die Belegung der Löcher der Trommel der Vereinzelungseinrichtung überwacht. Eine solche Kontrollvorrichtung kann für sämtliche Lochreihen gemeinsam wirken, beispielsweise auf dem Wege einer Überwachung des in dem Gehäuse herrschenden Druckes; denn wenn eines der Löcher (oder gar mehrere von ihnen) nicht belegt sind, erfolgt eine substantielle Ausströmung von Druckluft über die Trommel mit dem Ergebnis eines Druckverlusts im Gehäuse. Andere Formen der Überwachung samt einer Überwachung der einzelnen Lochreihen (z.B. über zugeordnete Sensoren) sind ebenfalls möglich.

Auch ist, gemäß einer wiederum anderen bevorzugten Weiterbildung der vorliegenden Erfindung, vorteilhaft, wenn das Verschießen der einzelnen Ausbringeinheiten durch die jeweiligen Ausblasrohre überwacht wird. Besonders bevorzugt kommen in diesem Zusammenhang den einzelnen Ausblasrohren zugeordnete Druckaufnehmer zum Einsatz, die so empfindlich sind, dass sie bereits die beim Verschießen der Ausbringeinheiten durch das betreffende Ausblasrohr entstehenden (geringen) Druckimpulse registrieren. Die bei der Überwachung des Betriebs des Ausbringfahrzeugs gewonnenen Daten können gespeichert und überdies mit Zeit- und/oder Positionsdaten (GPS-Daten) verknüpft werden, um den Einsatz zu dokumentieren und zu protokollieren, nicht zuletzt auch als Grundlage für einen Nachweis der Behandlung (und deren Abrechnung) der jeweiligen Fläche.

Ebenfalls überwacht wird vorteilhafterweise der Füllstand des Vorratsbehälters für die Ausbringeinheiten, um auf diese Weise ein rechtzeitiges Nachfüllen zu begünstigen. Gerade bei großen zu behandelnden Flächen mit langen Bahnen ist unter Gesichtspunkten der Wirtschaftlichkeit weiterhin günstig, die Nähe zu einem Versorgungsfahrzeug für ein Befüllen des Vorratsbehälters auszunutzen, selbst wenn letzterer noch nicht vollständig entleert ist, wozu die Füllstandsanzeige nützlich ist. Dies vermeidet Lehrfahrten und steigert die Effizienz. Die alternative oder zusätzliche Überwachung des Füllstandes in dem Raum um die Trommel herum ist unter Aspekten der Betriebssicherheit vorteilhaft; denn eine Betriebsstörung (beispielsweise durch ein gestörtes Nachrutschen der Ausbringeinheiten zur Trommel aufgrund eines Fremdkörpers) wird hierdurch frühzeitig erkannt.

Gemäß einer bevorzugten Weiterbildung der vorliegenden Erfindung, welche die landgestützte Anwendung von hier in Rede stehenden Verfahrens betrifft, handelt es sich bei dem Ausbringfahrzeug um ein Landfahrzeug, das durch den Bestand fährt, wobei die seitwärts gerichteten Ausblasrohre im Bereich ihrer Mündungen seitwärts nach oben gerichtet sind. Auf diese Weise lassen sich wirtschaftlich besonders attraktive Arbeitsbreiten erzielen, z.B. auf 8m links und rechts vom Ausbringfahrzeug. Zwei einander benachbarte Bahnen des Ausbringfahrzeugs können auf diese Weise einen Abstand von 24m zueinander einhalten, ohne eine gleichmäßige, flächendeckende Ausbringung in dem Bestand zu gefährden. Statt einer landgestützten Ausbringung kommt allerdings auch eine luftgestützte Ausbringung mittels eines Luftfahrzeugs, welches mit dem erfindungsgemäßen Ausbringgerät ausgestattet ist, in Betracht, beispielsweise bei der Bekämpfung von Schädlingen auf für Landfahrzeuge schwer zugänglichen Flächen.

Gemäß einer anderen bevorzugten Weiterbildung der vorliegenden Erfindung wird die Taktung des Verschießens der einzelnen Ausbringeinheiten automatisch in Abhängigkeit von der Fortbewegungsgeschwindigkeit des Ausbringfahrzeugs über dem Boden des Bestands gesteuert. Auf diese Weise wird sichergestellt, dass auch bei sich ändernden Geschwindigkeit der Fortbewegung des Ausbringfahrzeugs über der fraglichen Fläche die angestrebte Ausbringdichte (z.B. zwischen 75 und 200 Ausbringeinheiten pro Hektar) zuverlässig eingehalten wird. Gemäß einer insoweit besonders vorteilhaften Weiterbildung wird die Fortbewegungsgeschwindigkeit des Ausbringfahrzeugs über dem Boden des Bestands unter Verwendung von GPS-Daten ermittelt. Dies ist insbesondere bei einer luftgestützten Ausbringung sehr vorteilhaft, um eine Verfälschung der Ausbringdichte durch Windeinflüsse zu minimieren. Alternativ und/oder additiv lässt sich die Taktung des Verschießens der einzelnen Ausbringeinheiten manuell über ein entsprechendes Bedienelement an einem dem Fahrzeugführer zugänglichen Steuerpult einstellen. Auf diese Weise kann beispielsweise der Fahrer des Ausbringfahrzeugs gezielt die Dosierung erhöhen bzw. reduzieren in Abhängigkeit von der individuellen Wahrnehmung beim Befahren bzw. Überfliegen des Bestands, so dass beispielsweise auf ausgeprägte erkennbare Schädlingsschwerpunkte unmittelbar mit einer erhöhten Ausbringdichte reagiert werden kann.

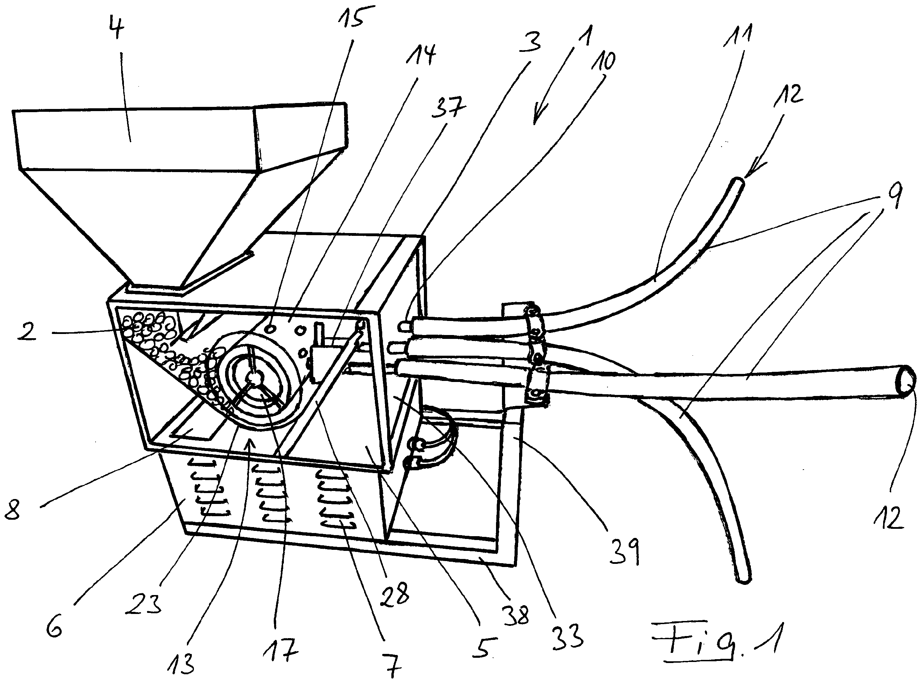

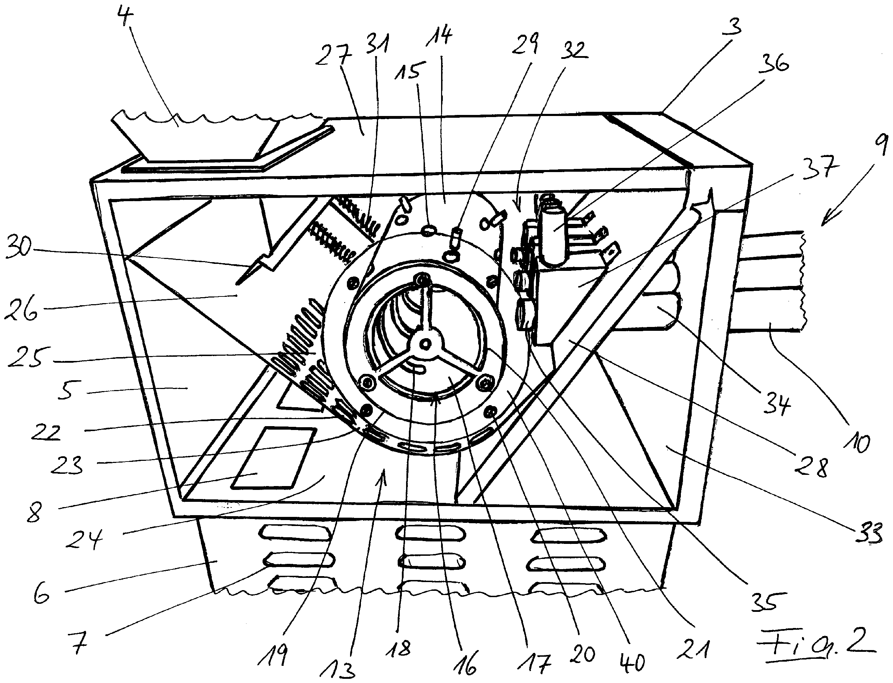

Im Folgenden wird die vorliegende Erfindung anhand eines in der Zeichnung veranschaulichten bevorzugten Ausführungsbeispiels näher erläutert. Dabei zeigen die Figuren 1 und 2 ein im Rahmen der vorliegenden Erfindung einsetzbares Ausbringgerät aus unterschiedlichen Perspektiven, verschieden detailliert und einmal mit und einmal ohne Ausbringkugeln.

Das in der Zeichnung gezeigte Ausbringgerät 1, mit dem ein Landfahrzeug wie insbesondere ein landwirtschaftlicher Schlepper ausgestattet werden kann, ist hergerichtet zum großflächigen Ausbringen von im Wesentlichen kugelförmigen Ausbringeinheiten (hier vereinfachend: "Ausbringkugeln"), welche zur biologischen Schädlingsbekämpfung geeignete Nützlingsorganismen enthalten, in einen land-, forst- oder gartenwirtschaftlichen Bestand durch einzeln getaktetes Verschießen mittels Druckluft. Im dargestellten Fall weisen die zu verschießenden Ausbringkugeln 2 einen mittleren Durchmesser von etwa 20mm und eine mittleren Dichte von etwa 0,25g/cm³ auf. Sie können fertigungsbedingt einen umlaufenden Rand ("Äquator") aufweisen, längs dessen zwei Halbschalen zusammengefügt sind, wobei die Halbschalen durchaus in nennenswerter Weise von einer mathematisch exakten Halbkugelform abweichen können. Insoweit dürfen die Begriffe "im Wesentlichen kugelförmig" und "Ausbringkugel" nicht eng ausgelegt werden.

Das Ausbringgerät 1 weist ein vorliegend etwa kubisches Gehäuse 3 auf, an das oben ein im Wesentlichen druckdicht verschlossener trichterförmiger Vorratsbehälter 4 für die Ausbringkugeln 2 druckdicht angeschlossen ist. Zur Veranschaulichung der Einbauten des Ausbringgeräts 1 ist die dem Betrachter der Zeichnung zugewandte Seitenwand 5 als durchsichtig dargestellt, was im Übrigen vorteilhafterweise durchaus der praktischen Ausführung entsprechen kann, um zu gewährleisten, dass die korrekte Funktion des Ausbringgeräts jederzeit beobachtet werden kann.

Unterhalb des Gehäuses 3 ist an dieses ein Gebläsekasten 6 angeschlossen. In diesem ist ein Druckluftgebläse untergebracht. Dieses saugt durch die Luftschlitze 7 Luft in den Gebläsekasten 6 ein, welche durch die beiden bodenseitig am Gehäuse 3 vorgesehenen Drucklufteinlässe 8 in das Gehäuse 3 einströmt. Das Druckluftgebläse ist im Zusammenwirken mit den weiteren Komponenten des Ausbringgeräts, d.h. insbesondere der Anzahl und den Querschnitten der Ausblasrohre (s.u.), so bemessen, dass sich im Gehäuse 3 ein Überdruck in der Größenordnung von etwa 10mbar einstellt. Bei der abgebildeten Ausführungsform mit drei Ausblasrohren 9, die auf Ausbringkugeln 2 mit einem mittleren Durchmesser von 20mm abgestimmt sind, kommt ein Druckluftgebläse (beispielsweise ein Doppel-Radialgebläse) zum Einsatz, dass den Überdruck von etwa 10mbar bei einem Luftfördervolumen von etwa 300m³/h bereitstellt.

An das Gehäuse des Ausbringgeräts sind drei Ausblasrohre 9, welche einen Innendruchmesser von etwa 23mm aufweisen, luftdicht angeschlossen, durch welche hindurch die Ausbringkugeln 2 mittels der Druckluft verschossen werden. Die Ausblasrohre 9 weisen jeweils einen ersten, starren Abschnitt 10 und einen zweiten, flexiblen Abschnitt 11 auf. Bei zweien der drei Ausblasrohre 9 verlaufen die zweiten Abschnitte 11 seitwärts und nach oben gekrümmt, so dass die Mündungsöffnungen 12 dieser Ausblasrohre seitwärts nach oben gerichtet sind, und zwar zu beiden Seiten des mit dem Ausbringgerät 1 ausgestatten Fahrzeugs. Das dritte, mittige Ausblasrohr 9 ist demgegenüber im Bereich seiner Mündung nach unten gerichtet, und zwar in die Längsrichtung des Ausbringfahrzeugs weisend. Für die Ausstattung eines als Ausbringfahrzeug dienenden Luftfahrzeugs wären demgegenüber die beiden äußeren Ausblasrohre 9 - bei im Wesentlichen unveränderter Orientierung zu den beiden Seiten - mündungsseitig typischerweise nach unten, d.h. seitwärts nach unten zu richten.

Zum getakteten, einzelnen Verschießen der Ausbringkugeln 2 durch die Ausblasrohre 9 hindurch verfügt das Ausbringgerät 1 über eine in seinem Gehäuse 3 untergebrachte Vereinzelungseinrichtung 13, mittels derer die Ausbringkugeln 2 einzeln und getaktet den - ständig mit Druckluft durchströmten - Ausblasrohren 9 zugeführt werden. Die Vereinzelungseinrichtung 13 umfasst eine (mittels eines nicht gezeigten Elektromotors) um ihre Achse drehend angetriebene Trommel 14 (in der Ansicht gemäß der Zeichnung im Uhrzeigersinn drehend), welche drei sich in Umfangsrichtung erstreckende Reihen von jeweils vier gleichmäßig zueinander beabstandeten Löchern 15 mit einem Durchmesser von etwa 12mm aufweist. Die Lochreihen fluchten zu den ersten Abschnitten 10 der Ausblasrohre 9. Die Löcher 15 in den einzelnen Lochreihen der Trommel 14 sind dabei zueinander in Umfangsrichtung versetzt angeordnet. Stirnseitig ist die Trommel 14 gegenüber dem Gehäuse 3, d.h. dessen einander gegenüberliegenden Seitenwänden 5, zwischen denen die Trommel 14 sich erstreckt, abgedichtet. Innen in der Trommel 14 ist eine an dieser anliegende, nicht-drehende Abdeckeinrichtung 16 in Form einer ruhenden, aus Kunststoff bestehenden Innentrommel 17 angeordnet. Diese ist als Steuerhülse ausgeführt, indem sie sich in Umfangsrichtung über einen Teil des Umfangs erstreckende, zu den Lochreihen der Trommel 14 fluchtende Schlitze 18 aufweist. In jenem Winkelbereich, über den die Schlitze 18 sich nicht erstrecken, verschließt die Innentrommel 17 die Löcher 15 der Trommel 14 an deren Innenseite.

Die Trommel 14 und die Innentrommel 17 sind Teil eines in Art einer Kartusche ausgeführten Einsatzes, an dem stirnseitig ein (wiederum transparent gezeichneter) Montagering 40 angeordnet ist. Die dem Betrachter der Zeichnung zugewandte Seitenwand 5 des Gehäuses 1 weist einen Gehäusedurchbruch 19 auf, durch welchen hindurch der Einsatz in das Gehäuse 3, ohne dass dieses hierfür zerlegt werden müsste, eingesetzt werden kann, und zwar so weit, bis der Montagering 40 um den Gehäusedurchbruch 19 herum außen an der betreffenden Seitenwand 5 anliegt. Zwischen Montagering 40 und Seitenwand 5 des Gehäuses 3 ist eine Dichtung angeordnet; und der Montagering 40 wird mittels Schrauben 20 an der Seitenwand 5 des Gehäuses 3 fixiert. Das Innere der Trommel 14 bzw. der Innentrommel 17 ist durch die zentrale Öffnung 21 des Montageringes 40 hindurch belüftet, so dass dort Umgebungsdruck herrscht.

Unterhalb der Trommel 14 ist innerhalb des Gehäuses 3 ein - eine Vielzahl von eng zueinander angeordneten Langlöchern 22 aufweisendes - Lochblech 23 angeordnet. Der Raum 24 unterhalb des Lochblechs 23 bildet einen Druckluft-Ausgleichs- und -Beruhigungsraum. Die Druckluft durchströmt das Lochblech 23 vom Ausgleichs- und - Beruhigungsraum in Richtung auf den Raum 25, in dem sich die Trommel 14 und die Ausbringkugeln 2 befinden. Das Lochblech 23 hält zu der Trommel 14 einen Abstand ein, der größer ist als der mittlere Durchmesser der Ausbringkugeln 2, so dass letztere zwischen das Lochblech 23 und die Trommel 14 gelangen können. Das Lochblech 23 geht, in der Zeichnung links dargestellt, in ein geneigtes Zuführblech 26 über. Über dieses werden Ausbringkugeln 2, die aus dem Vorratsbehälter 4 durch einen Durchbruch in der Oberwand 27 des Gehäuses 3 in dieses hinein gelangen, der Trommel 14 zugeführt. An seinem gegenüberliegenden, in der Zeichnung rechts gezeigten Randabschnitt ist das Lochblech 23 an einer geneigten Trennwand 28 befestigt. Auf der Trommel 14 sind nach außen abstehende stift- oder zapfenförmige Rührvorsprünge 29 angebracht, welche - zusätzlich zu der wirbelbettartigen Verwirbelung der Ausbringkugeln 2 oberhalb des Lochblechs 23 - für eine ständige Bewegung der Ausbringkugeln 2 in der Umgebung der Trommel 14 sorgen, was ein Anlagern der Ausbringkugeln 2 an der Trommel 14 an deren Löchern 15 begünstigt.

Benachbart zu der Öffnung, durch welche hindurch die Ausbringkugeln 2 aus dem Vorratsbehälter 4 in das Gehäuse 3 gelangen, ist ein Leit- und Abschirmblech 30 vorgesehen, welches - über den verbleibenden Spalt zu dem Zuführblech 26 - den "Füllstand" der Ausbringkugeln 2 in dem Raum um die Trommel 14 herum einstellt. Von dem Leit- und Abschirmblech 30 springen in Richtung auf die Trommel 14 zwei federnd nachgiebige Abstreifer 31 vor. Diese sind jeweils zwischen zwei Lochreihen angeordnet und unterbinden, dass zwei zunächst an einem Loch 15 angesaugte Ausbringkugeln (sog. "Doppelbelegung") gemeinsam dem betreffenden Ausblasrohr 9 zugeführt werden.

Eingangseitig ist den drei Ausblasrohren 9 jeweils ein Verstellmechanismus 32 zugeordnet, mittels dessen eine Übergabe von Ausbringkugeln 2 an das betreffende Ausblasrohr 9 unterbunden werden kann. Hierzu sind die ersten Abschnitte 10 der Ausblasrohre 9 zweiteilig ausgeführt. Jeweils ist ein sich durch die Stirnwand 33 des Gehäuses 3 erstreckender erster Teil 34 an der Stirnwand 33 sowie der Trennwand 28 fixiert. Ein der Trommel 14 gegenüberstehender zweiter Teil 35 der ersten Abschnitte 10 der Ausblasrohre 9 ist demgegenüber jeweils vertikal verschiebbar zwischen einer unteren Stellung, in der er mit dem jeweils zugeordneten ersten Teil 34 fluchtet und einen Abstand von nur etwa 25mm zu der Trommel 14 einhält, und einer oberen, nicht mit dem jeweils zugeordneten ersten Teil 34 fluchtenden Stellung. In letzterer sind die zweiten Teile 35 der ersten Abschnitte 10 der Ausblasrohre 9 nicht durchströmt. Zudem stehen sie der Trommel 14 nicht so dicht gegenüber wie in der unteren Stellung. Im Ergebnis werden die an der Trommel 14 haftenden Ausbringkugeln 2 nicht an das - insoweit jeweils "außer Betrieb" gesetzte - Ausblasrohr 9 übergeben. Löst sich die jeweilige Ausbringkugel 2 in der Stellung der Trommel 14, in der das betreffende Loch 15 der Trommel 14 durch die Innentrommel 15 (Steuerhülse) verschlossen wird, von der Trommel 14 ab, so fällt sie auf das Lochblech 23 herunter. Wesentlich ist in diesem Zusammenhang, dass bei einem solchen "Außerbetriebsetzen" eines Ausblasrohres 9 (oder mehrerer Ausblasrohre 9) die Luftdurchströmung durch das betreffende Ausblasrohr 9 (bzw. die betreffenden Ausblasrohre 9) nicht unterbunden sondern vielmehr - zumindest weitgehend - aufrechterhalten wird, um eine durch veränderte Druck- und/oder Strömungsverhältnisse begründete Rückwirkung auf die weiterhin in Betrieb befindlichen Ausblasrohre 9 zu unterbinden.

Der beschriebenen Verschiebung der zweiten Teile 35 der ersten Abschnitte 10 der Ausblasrohre 9 nach oben bzw. unten dienen Aktoren 36, die auf Konsolen 37 angebracht sind, welche ihrerseits auf der Trennwand 28 montiert sind. Die Aktoren 36 sind von einem für den Fahrer des Ausbringfahrzeugs zugänglichen Bediengerät aus betätigbar. Von jenem Bediengerät aus ist weiterhin auch die Drehzahl der Trommel 14 manuell veränderbar, um aktiv Einfluss nehmen zu können auf die Ausbringdichte der Ausbringkugeln 2 im Bestand. Im Übrigen wird - durch automatische Verstellung der Drehgeschwindigkeit der Trommel 14 - automatisch die Taktung des Verschießens der einzelnen Ausbringkugeln in Abhängigkeit von der Fortbewegungsgeschwindigkeit des Ausbringfahrzeugs über dem Boden des Bestands gesteuert, so dass die eingestellte Ausbringdichte aufrechterhalten wird. Die Fortbewegungsgeschwindigkeit des Ausbringfahrzeugs über dem Boden des Bestands kann dabei insbesondere unter Verwendung von GPS-Daten ermittelt werden.

Erkennbar ist in Fig. 1 schließlich ein Montagerahmen 38, auf dem die Einheit aus dem Gebläsekasten 6 und dem Gehäuse 3 aufgebaut ist und an welchen eine die Ausblasrohre 9 entfernt vom Gehäuse 3 unterstützende Stützstruktur 39 angebracht ist.

## Patentansprüche

1. Ausbringfahrzeug für das großflächige Ausbringen von im Wesentlichen kugelförmigen, zur biologischen Schädlingsbekämpfung geeignete Nützlingsorganismen enthaltenden Ausbringeinheiten mit einem mittleren Durchmesser zwischen 15mm und 30mm und einer mittleren Dichte zwischen 0,08g/cm³ und 1,5g/cm³ in einen land-, forst- oder gartenwirtschaftlichen Bestand, indem das Ausbringfahrzeug in Bahnen über den Boden des Bestands bewegt wird und die Ausbringeinheiten einzeln getaktet mittels Druckluft aus mehreren seitwärts gerichteten, frei mündenden Ausblasrohren (9) zu beiden Seiten des Fahrzeugs verschossen werden, **gekennzeichnet durch** die folgenden Merkmale :
das Ausbringfahrzeug ist mit einem Ausbringgerät (1) ausgestattet, welches ein Gehäuse (3), einen Vorratsraum für die Ausbringeinheiten und mehrere Ausblasrohre (9) für die auszubringenden Ausbringeinheiten umfasst;
die Ausblasrohre (9) sind strömungstechnisch an den Innenraum des Gehäuses (3) angeschlossen;
zumindest zwei der Ausblasrohre (9) münden zu unterschiedlichen Seiten des Fahrzeugs orientiert;
es ist mindestens ein Druckluftgebläse vorgesehen, welches geeignet ist, aus der Umgebung angesaugte Luft kontinuierlich **durch** das Gehäuse (3) und mit mindestens 20m/s **durch** die Ausblasrohre (9) zu fördern und dabei in dem Gehäuse (3) einen Überdruck von mindestens 5mbar zu erzeugen;
das Ausbringgerät (1) weist eine Einrichtung (13) zum Vereinzeln der Ausbringeinheiten und zu deren getakteter Zufuhr zu den Ausblasrohren (9) auf;
die Vereinzelungseinrichtung (13) umfasst dabei eine drehend angetriebene, stirnseitig gegenüber dem Gehäuse (3) abgedichtete, innen dem Umgebungsdruck oder einem zwischen diesem und dem Gehäuse-Überdruck liegenden Druckniveau ausgesetzte Trommel (14) mit sich in Umfangsrichtung erstreckenden Reihen von zueinander beabstandeten Löchern (15) und eine innen an der Trommel anliegende, nicht-drehende Abdeckeinrichtung (16) für das Verschließen der Löcher (15) über einen vorgegeben Winkelbereich, wobei die Löcher (15) in den einzelnen Lochreihen der Trommel (14) zueinander in Umfangsrichtung versetzt sind.

2. Ausbringfahrzeug nach Anspruch 1, **dadurch gekennzeichnet, dass** die Abdeckeinrichtung (16) eine ruhende Innentrommel (17) in Form einer Steuerhülse umfasst, die sich über einen Teil des Umfangs in Umfangsrichtung erstreckende, zu den Lochreihen der Trommel fluchtende Schlitze (18) aufweist.

3. Ausbringfahrzeug nach einem der Ansprüche 1 bis 2, **dadurch gekennzeichnet, dass** die Löcher (15) einen Durchmesser aufweisen, der zwischen 45% und 75% des mittleren Durchmessers der Ausbringeinheiten liegt.

4. Ausbringfahrzeug nach einem der Ansprüche 1 bis 3, **dadurch gekennzeichnet, dass** unterhalb der Trommel (14) innerhalb des Gehäuses (3) ein vom Druckluftgebläse her mit Luft durchströmtes Lochblech (23) angeordnet ist.

5. Ausbringfahrzeug nach einem der Ansprüche 1 bis 4, **dadurch gekennzeichnet, dass** die Drehzahl der Trommel (14) von einem Steuergerät aus veränderbar ist.

6. Ausbringfahrzeug nach einem der Ansprüche 1 bis 5, **dadurch gekennzeichnet, dass** die Trommel (14) durch einen Gehäusedurchbruch (19) hindurch ohne Zerlegen des Gehäuses (3) auswechselbar ist.

7. Ausbringfahrzeug nach einem der Ansprüche 1 bis 6, **dadurch gekennzeichnet, dass** auf der Trommel (14) nach außen abstehende Rührvorsprünge (29) angebracht sind.

8. Ausbringfahrzeug nach einem der Ansprüche 1 bis 7, **dadurch gekennzeichnet, dass** der Innendurchmesser der Ausblasrohre (9) zwischen 10% und 20% über dem mittleren Durchmesser der Ausbringeinheiten liegt.

9. Ausbringfahrzeug nach einem der Ansprüche 1 bis 8, **dadurch gekennzeichnet, dass** das Ausbringgerät (1) drei Ausblasrohre (9) aufweist, wobei ein mittiges Ausblasrohr mit einer in der Längsrichtung des Ausbringfahrzeugs weisenden Mündungsöffnung (12) vorgesehen ist.

10. Ausbringfahrzeug nach einem der Ansprüche 1 bis 9, **dadurch gekennzeichnet, dass** mindestens einem der Ausblasrohre (9) eingangsseitig ein von einem Bediengerät aus betätigbarer Verstellmechanismus (32) zugeordnet ist, mittels dessen eine Übergabe von Ausbringeinheiten an das betreffende Ausblasrohr (9) unterbunden werden kann.

11. Ausbringfahrzeug nach einem der Ansprüche 1 bis 10, **dadurch gekennzeichnet, dass** es sich um ein Landfahrzeug handelt, wobei die seitwärts weisenden Ausblasrohre (9) im Bereich ihrer Mündungen (12) seitwärts nach oben gerichtet sind, wobei das Ausbringgerät bevorzugt einen Montagerahmen (38) umfasst, an welchen weiterhin eine die Ausblasrohre (9) entfernt vom Gehäuse (3) unterstützende Stützstruktur (39) angebracht ist.

12. Ausbringfahrzeug nach einem der Ansprüche 1 bis 11, **dadurch gekennzeichnet, dass** eine Steuereinheit vorgesehen ist, welche die Taktung des Verschießens der einzelnen Ausbringeinheiten automatisch in Abhängigkeit von der Fortbewegungsgeschwindigkeit des Ausbringfahrzeugs über dem Boden des Bestands steuert, wobei das Ausbringfahrzeug zur Ermittlung der Fortbewegungsgeschwindigkeit des Ausbringfahrzeugs über dem Boden des Bestands bevorzugt mit einem GPS-Modul ausgestattet ist.

13. Ausbringfahrzeug nach einem der Ansprüche 1 bis 12, **dadurch gekennzeichnet, dass** den Ausblasrohren (9) Druckmessumformer zugeordnet sind, die zum Erfassen von beim Verschießen von Ausbringeinheiten in dem jeweiligen Ausblasrohr entstehenden Druckimpulsen geeignet sind.

## Claims

1. A spreading vehicle for spreading, over a large area, substantially spherical spreading units containing useful organisms suitable for biological pest control and having a mean diameter between 15 mm and 30 mm and a mean density between 0.08 g/cm³ and 1.5 g/cm³ in an agricultural, silvicultural or horticultural terrain by moving the spreading vehicle in tracks over the ground of the terrain and shooting the spreading units in an individually clocked manner by means of compressed air from a plurality of freely discharging delivery pipes (9) directed sidewards on both sides of the vehicle, **characterised by** the following features:
the spreading vehicle is equipped with a spreading apparatus (1), which comprises a housing (3), a storage space for the spreading units, and a plurality of delivery pipes (9) for the spreading units to be spread;
the delivery pipes (9) are fluidically connected to the inside of the housing (3);
at least two of the delivery pipes (9) are oriented so as to open out on different sides of the vehicle;
at least one compressed air blower is provided, which is suitable for continuously conveying air sucked in from the surroundings through the housing (3) and through the delivery pipes (9) at a rate of at least 20 m/s and at the same time to generate an overpressure in the housing (3) of at least 5 mbar;
the spreading device (1) has a means (13) for separating the spreading units and for feeding said units in a clocked manner to the delivery pipes (9);
the separating means (13) here comprises a drum (14), which is driven in rotation, is sealed off at the end faces with respect to the housing (3), is exposed internally to the ambient pressure or a pressure level lying between ambient pressure and the housing overpressure, said drum having rows, extending in the peripheral direction, of holes (15) distanced from one another and a non-rotating covering means (16), which bears internally against the drum, for closing the holes (15) over a specified angular range, wherein the holes (15) in the individual rows of holes in the drum (14) are offset in relation to one another in the peripheral direction.

2. The spreading vehicle according to claim 1, **characterised in that** the covering means (16) comprises a static internal drum (17) in the form of a control sleeve, which has slots (18) extending in the peripheral direction over part of the periphery and aligned with the rows of holes in the drum.

3. The spreading vehicle according to either one of claims 1 or 2, **characterised in that** the holes (15) have a diameter which is between 45% and 75% of the mean diameter of the spreading units.

4. The spreading vehicle according to ay one of claims 1 to 3, **characterised in that** a perforated plate (23) through which air passes from the compressed air blower is arranged beneath the drum (14) inside the housing (3).

5. The spreading vehicle according to any one of claims 1 to 4, **characterised in that** the rotational speed of the drum (14) can be changed from a control apparatus.

6. The spreading vehicle according to any one of claims 1 to 5, **characterised in that** the drum (14) can be replaced through a housing aperture (19) without dismantling the housing (3).

7. The spreading vehicle according to any one of claims 1 to 6, **characterised in that** outwardly protruding agitating protrusions (29) are mounted on the drum (14).

8. The spreading vehicle according to any one of claims 1 to 7, **characterised in that** the inner diameter of the delivery pipes (9) is between 10% and 20% greater than the mean diameter of the spreading units.

9. The spreading vehicle according to any one of claims 1 to 8, **characterised in that** the spreading apparatus (1) has three delivery pipes (9), wherein a central delivery pipe is provided with an outlet opening (12) pointing in the longitudinal direction of the spreading vehicle.

10. The spreading vehicle according to any one of claims 1 to 9, **characterised in that** at least one of the delivery pipes (9), on the inlet side, is allocated an adjustment mechanism (32), which can be actuated from an operating unit and by means of which a transfer of spreading units to the relevant delivery pipe (9) can be prevented.

11. The spreading vehicle according to any one of claims 1 to 10, **characterised in that** it is a land vehicle, wherein the delivery pipes (9) pointing sidewards are directed sidewards and upwardly in the region of their outlets (12), wherein the spreading apparatus preferably comprises a mounting frame (38) on which there is also mounted a supporting structure (39) supporting the delivery pipes (9) at a distance from the housing (3).

12. The spreading vehicle according to any one of claims 1 to 11, **characterised in that** a control unit is provided which controls the clocking of the shooting of the individual spreading units automatically depending on the speed of travel of the spreading vehicle over the ground of the terrain, wherein the spreading vehicle is preferably equipped with a GPS module in order to ascertain the speed of travel of the spreading vehicle over the ground of the terrain.

13. The spreading vehicle according to any one of claims 1 to 12, **characterised in that** the delivery pipes (9) are allocated pressure transducers, which are suitable for detecting pressure pulses created when shooting spreading units in the respective delivery pipes.

## Revendications

1. Véhicule d'épandage pour l'épandage sur grandes surfaces d'unités d'épandage sensiblement en forme de billes, contenant des organismes utiles adaptés pour la lutte biologique contre les parasites, avec un diamètre moyen compris entre 15 mm et 30 mm et une densité moyenne comprise entre 0,08 g/cm³ et 1,5 g/cm³ dans une réserve d'exploitation agricole, sylvicole ou horticole, en ce que le véhicule d'épandage est déplacé sur des pistes sur les sols de la réserve et en ce que les unités d'épandage sont projetées de façon individuellement cadencée au moyen d'air comprimé à partir de plusieurs tuyaux d'expulsion par soufflage (9) orientés latéralement, débouchant librement, des deux côtés du véhicule,
**caractérisé par** les caractéristiques suivantes :
le véhicule d'épandage est équipé d'un appareil d'épandage (1) comprenant un boîtier (3), un espace de stockage pour les unités d'épandage et plusieurs tuyaux d'expulsion par soufflage (9) pour les unités d'épandage à épandre ;
les tuyaux d'expulsion par soufflage (9) étant raccordés par technique des fluides à l'espace intérieur du boîtier (3) ;
au moins deux des tuyaux d'expulsion par soufflage (9) débouchant de façon orientée vers des côtés différents du véhicule ;
au moins une soufflante pneumatique étant prévue, laquelle convient pour véhiculer en continu de l'air aspiré de l'environnement à travers le boîtier (3) et avec au moins 20m/s à travers les tuyaux d'expulsion par soufflage (9) en générant ce faisant dans le boîtier (3) une surpression d'au moins 5 mbars ;
l'appareil d'épandage (1) présentant un dispositif (13) pour la séparation des unités d'épandage et pour leur amenée cadencée aux tuyaux d'expulsion par soufflage (9) ;
le dispositif de séparation (13) comprenant alors un tambour (14) entraîné rotativement, étanchéifié du côté frontal par rapport au boîtier (3), exposé à l'intérieur à la pression environnante ou à un niveau de pression situé entre celle-ci et la surpression du boîtier, avec des rangées de trous (15) espacés entre eux s'étendant en direction périphérique et un dispositif de couverture (16) non rotatif reposant à l'intérieur contre le tambour pour la fermeture des trous (15) sur une plage angulaire prescrite, dans lequel les trous (15) sont décalés entre eux en direction périphérique dans les rangées individuelles de trous du tambour (14).

2. Véhicule d'épandage selon la revendication 1, **caractérisé en ce que** le dispositif de couverture (16) comprend un tambour intérieur (17) au repos sous la forme d'un manchon de commande qui présente des fentes (18) en affleurement avec les rangées de trous du tambour s'étendant sur une partie de la périphérie en direction périphérique.

3. Véhicule d'épandage selon l'une des revendications 1 à 2, **caractérisé en ce que** les trous (15) présentent un diamètre compris entre 45% et 75% du diamètre moyen des unités d'épandage.

4. Véhicule d'épandage selon l'une des revendications 1 à 3, **caractérisé en ce qu'**en-dessous du tambour (14), à l'intérieur du boîtier (3), une tôle perforée (23) traversée par de l'air provenant de la soufflante pneumatique est disposée.

5. Véhicule d'épandage selon l'une des revendications 1 à 4, **caractérisé en ce qu'**on peut faire varier la vitesse de rotation du tambour (14) à partir d'un appareil de commande.

6. Véhicule d'épandage selon l'une des revendications 1 à 5, **caractérisé en ce que** le tambour (14) peut être changé à travers une brèche du boîtier (19) sans démonter le boîtier (3).

7. Véhicule d'épandage selon l'une des revendications 1 à 6, **caractérisé en ce que** des saillies de malaxage (29) dépassant vers l'extérieur sont aménagées sur le tambour (14).

8. Véhicule d'épandage selon l'une des revendications 1 à 7, **caractérisé en ce que** le diamètre intérieur des tuyaux d'expulsion par soufflage (9) est supérieur de l'ordre de 10% à 20% au diamètre moyen des unités d'épandage.

9. Véhicule d'épandage selon l'une des revendications 1 à 8, **caractérisé en ce que** l'appareil d'épandage (1) présente trois tuyaux d'expulsion par soufflage (9), dans lequel un tuyau d'expulsion par soufflage du milieu avec une ouverture d'embouchure (12) orientée en direction longitudinale du véhicule d'épandage est prévu.

10. Véhicule d'épandage selon l'une des revendications 1 à 9, **caractérisé en ce que** l'on associe à au moins l'un des tuyaux d'expulsion par soufflage (9), du côté d'entrée, un mécanisme de réglage (32) actionnable par un organe de commande, avec lequel mécanisme on peut supprimer une transmission d'unités d'épandage au tuyau d'expulsion par soufflage (9) concerné.

11. Véhicule d'épandage selon l'une des revendications 1 à 10, **caractérisé en ce qu'**il s'agit d'un véhicule agricole, dans lequel les tuyaux d'expulsion par soufflage (9) orientés latéralement sont orientés latéralement vers le haut dans la zone de leurs embouchures (12), dans lequel l'appareil d'épandage comprend de préférence un cadre de montage (38) sur lequel une structure de support (39) supportant les tuyaux d'expulsion par soufflage (9) en éloignement du boîtier (3) est en outre aménagée.

12. Véhicule d'épandage selon l'une des revendications 1 à 11, **caractérisé en ce que** l'on prévoit une unité de commande, laquelle commande automatiquement la cadence de fermeture des unités d'épandage individuelles en fonction de la vitesse de déplacement du véhicule d'épandage sur le sol de la réserve, dans lequel le véhicule d'épandage est de préférence équipé d'un module GPS pour la détermination de la vitesse de déplacement du véhicule d'épandage sur le sol de la réserve.

13. Véhicule d'épandage selon l'une des revendications 1 à 12, **caractérisé en ce que** des capteurs de pression sont associés aux tuyaux d'expulsion par soufflage (9), lesquels conviennent pour la détection d'impulsions de pression se formant dans le tuyau d'expulsion par soufflage respectif lors de la fermeture d'unités d'épandage.
